Europäisches Patentamt

**European Patent Office**

**Office européen des brevets**

(19)

(11) Publication number : **0 130 162**
**B1**

(12)

# EUROPEAN PATENT SPECIFICATION

(45) Date of publication of patent specification :
09.09.87

(51) Int. Cl.⁴ : **B 01 J 13/02,** A 61 K  9/50

(21) Application number : **84850184.7**

(22) Date of filing : **14.06.84**

(54) **Small particle formation and encapsulation.**

(30) Priority : **22.06.83 US 506599**

(43) Date of publication of application :
**02.01.85 Bulletin 85/01**

(45) Publication of the grant of the patent :
**09.09.87 Bulletin 87/37**

(84) Designated contracting states :
**AT BE CH DE FR GB IT LI LU NL SE**

(56) References cited :
**DE-A- 1 542 262**
**DE-A- 3 013 839**
**US-A- 3 666 678**
**CHEMICAL ABSTRACTS, vol. 99, July 1983, Nr. 4, page 339, Nr. 28007a, COLUMBUS OHIO (US)**
**The file contains technical information submitted after the application was filed and not included in this specification**

(73) Proprietor : **THE OHIO STATE UNIVERSITY RESEARCH FOUNDATION**
**1314 Kinnear Road**
**Columbus Ohio 43212 (US)**

(72) Inventor : **Frank, Sylvan Gerald**
**1910 Wyandotte Road**
**Columbus Ohio 43212 (US)**
Inventor : **Brodin, Arne Folke**
**Gillestigen 29**
**S-151 52 Södertälje (SE)**
Inventor : **Chen, Chih-Ming James**
**414 Deerfield Road, Apt 4**
**East Syracuse New York 13057 (US)**
Inventor : **Panthuvanich, Siriporn**
**500 W. 12th Avenue**
**Columbus Ohio 42210 (US)**

(74) Representative : **Wurm, Bengt Runio et al**
**Patent and Trade Mark Department AB ASTRA**
**S-151 85 Södertälje (SE)**

EP 0 130 162 B1

**0 130 162**

## Description

The present invention is concerned with the simultaneous formation and encapsulation of small particles from aqueous solutions of compounds whose solubility is greater at a first pH than at a second pH. The process is preferably used to prepare a readily soluble encapsulated pharmaceutically active compound.

## Background of the invention

From a pharmaceutical point of view, the smaller the particle size of a relatively insoluble drug the greater is its rate of solution and as a rule, the greater is its bioavailability (J. H. Fincher, J. Pharm. Sci., 57, 1825 (1968)). To this end, small particles are conventionally formed by mechanical subdivision of bulk matter or by aggregation of small molecules of ions (D. J. Shaw, « Introduction to Colloid and Surface Chemistry », 3rd Ed., Butterworths, London, 1980, Chapter 1). The production and applications of microcapsules for medical and technical use have been extensively reviewed (L. A. Luzzi, J. Pharm. Sci., 59, 1367 (1970) ; A. Kondo, « Microcapsule Processing and Technology », Marcel Dekker, New York (1979) ; J. E. Vandegaer, « Microencapsulation : Processes and Applications », Plenum Press, New York (1976) ; J. R. Nixon, « Microencapsulation », Marcel Dekker, New York (1976) ; J. A. Bakan and J. L. Anderson, in « The Theory and Practice of industrial Pharmacy », Second Ed. (Ed. L. Lachman, et al.), Lea & Febiger, Philadelphia, 1976, p. 420 ; M. H. Gutcho, « Microcapsules and Microencapsulation Techniques », Noyes Data Corp., New Jersey (1976)).

The preparation of small particles by a procedure wherein a drug is dissolved in a basic solvent and then neutralized with an acid or alternatively wherein a drug is dissolved in an acidic solvent and then neutralized with a base is disclosed in DE 3013839.

## Summary of the invention

A method has now been found which involves the formation of small core particles of a compound from solution and the concurrent encapsulation of the core particles in a coacervate of the encapsulating material as the pH of the system is altered. This process of encapsulation in a natural or synthetic polymer protects and stabilizes the active core compound. The new method for encapsulating weakly acidic or weakly basic organic compounds whose solubility in water decreases from a first pH to a second pH which comprises :

(a)  dissolving said compound in the case of a weakly acidic compound in water in the presence of sufficient base to raise the pH to said first pH and at least 2 pH units above the pKa of the compound ; and in the case of a weakly basic compound dissolving the compound in water in the presence of sufficient acid to lower the pH to said first pH and at least 2 pH units below the pKa of the compound ; together with an encapsulating material and an electrolyte which is effective but present in an amount just insufficient, to cause coacervation of the encapsulating material without interacting with it ; and

(b)  stirring and titrating the solution with an acid or basic titrant effective to raise or lower the pH of said solution to said second pH to cause the concurrent precipitation of the compound as small particles and formation of a coacervate of the encapsulating material ; and

(c)  gelling the encapsulating material.

In this process coacervation of the encapsulating material is believed to result from the addition of the acid or base during titration step (b), which amounts to sufficient additional electrolyte, when taken together with the electrolyte initially present to cause coacervation.

In an alternate embodiment, a suitable wetting agent or surfactant such as cetyltrimethylammonium bromide or sodium lauryl sulfate may also be used in step (a) of this process and the pH should be adjusted above or below the pKa depending on whether the compound is weakly acidic or basic, preferably at least 2 pH units below or above the pKa.

Suitable pharmaceutically active compounds whose solubility in water decreases at a first pH to a second pH are, for example, bacampicillin, griseofulvin, indomethacin, sodium sulfadiazine, erythromycin, theophylline, salicylic acid, acetylsalicylic acid, chlorzoxazone, lidocaine and alaproclate.

A suitable encapsulating material which will form a coacervate is, for example, gelatin (preferably of the type B ; acid processed), methylcellulose, sodium carboxymethylcellulose, cellulose acetate phthalate and polyvinylpyrrolidone. A suitable electrolyte which is effective to cause coacervation of the encapsulating material without interacting with it is, for example, sodium sulfate solution, preferably a 5-30 % aqueous solution which may also contain a suitable cosolvent, for example, an alcohol at about 0-10 %. The compound, encapsulating material, wetting agent and electrolyte can be combined in step (a) in ratios of about (0.1-6) : (0.1-4) : (0.1-10) : (0.4-48).

If the compound is weakly acidic, the titration in step (b) can be carried out, for example, with hydrochloric acid or acetic acid to a pH of about 4.3-4.5. If the compound is basic, sodium or potassium

2

hydroxide can be used to a pH of about 8-10. If necessary, the temperature can be adjusted during the titration. The gelling of the encapsulating material can be achieved by treatment of the encapsulating material with cold (5 °C) Na₂SO₄ solution. If polyvinylpyrrolidone is used as the encapsulating material gelling can also be achieved by a number of other methods, for example :

1. application of heat (to 60 °C) ;
2. addition of hydrochloric acid, 0.05N-1. ON (10 ml 0.1M HCl/ml) to the mixture to be gelled ;
3. application of heat (40-45 °C) plus addition of sodium sulfate solution ;
4. application of heat plus addition of hydrochloric acid ;
5. application of heat (40-45 °C) plus addition of hydrochloric acid and sodium sulfate.

Once formed, the microcapsules can be collected by conventional means, for instance, by centrifugation. The encapsulated particles formed by this process are less than 100 μm, preferably less than 10 μm ; and the core particles are less than 25 μm, preferably less than 1 μm.

Detailed description of the invention

According to one embodiment, the process comprises the following steps which are carried out at a temperature above 35 °C, preferably 40-45 °C.

(a) dissolving a suitable pharmaceutically active compound in a solution of sodium sulfate containing ethyl alcohol, or another similar water miscible higher alcohols, for example, propanol or butanol ;
(b) adding a gelatin solution to the solution from step a ; and
(c) titrating the solution obtained in step (b) with a suitable acid or base titrant while keeping the solution under constant agitation which results in a suspension of encapsulated pharmaceutically active small particles and coacervation of the gelatin.

The suspension is then poured into cold sodium sulfate solution and stirred at the temperature of an ice bath. This procedure causes « hardening » of the liquid gelatin shell of the microcapsules. The microcapsules are then collected, for instance, by centrifugation. The ratio of pharmaceutically active compound to sodium sulfate to ethyl alcohol is, for example, 1 : 7 : 1. The gelatin solution should preferably be prepared from type B (acid processed) gelatin, and of a pharmaceutical grade. The gelatin should be added as a 2-10 % (w/w) solution, preferably a 5 % (w/w) solution.

Example 1

An aqueous solution consisting of 0.5 g sodium sulfadiazine, 0.5 ml ethyl alcohol, 13 ml of 20 % sodium sulfate and 20 ml of 5 % gelatin (type B : acid processed) was titrated, while under constant agitation with a magnetic stirrer, with 18.4 ml of 0.1N hydrochloric acid solution. This procedure resulted in a white suspension of microencapsulated sulfadiazine particles. The suspension was then stirred for an additional 15 minutes, following which it was poured into 200 ml of cold (5 °C) 7 % sodium sulfate solution, and stirred for 30 minutes at ice-bath temperature. This procedure caused gelling of the liquid gelatin shell of the microcapsules. The entire process was monitored by observation of samples in the optical microscope. The microcapsules were of assymetric appearance and of a size less than 10 μm.

A schematic diagram of the preparation of the microcapsules according to Example 1 is illustrated below :

/

```
┌─────────────────────────────────────────────────────────────┐
│ Dissolve sodium sulfadiazine (~4%) in 20% sodium             │
│ sulfate solution containing ethyl alcohol.                   │
│                                                              │
└─────────────────────────────────────────────────────────────┘
                              │
                              ↓
┌─────────────────────────────────────────────────────────────┐
│                    Add 5% gelatin solution                   │
└─────────────────────────────────────────────────────────────┘
                              │
                              ↓
┌─────────────────────────────────────────────────────────────┐
│ Form sulfadiazine particles by acidification                 │
│ (simultaneously encapsulating with gelatin).                 │
└─────────────────────────────────────────────────────────────┘
                              │

        Steps above this line performed at 40-45°C,
          which is above the gelling point of gelatin (35°).
                              │
                              ↓
┌─────────────────────────────────────────────────────────────┐
│ Gel the encapsulating material by pouring the                │
│ suspension into cold (5°C) sodium sulfate solution.          │
└─────────────────────────────────────────────────────────────┘
                              │
                              ↓
┌─────────────────────────────────────────────────────────────┐
│ Collect the microcapsules by centrifugation.                 │
└─────────────────────────────────────────────────────────────┘
```

According to a second embodiment of the invention. The process comprises the following steps which are carried out at 40-45 °C.

(a) mixing solutions of a suitable pharmaceutically active compound, cetyltrimethylammonium bromide and gelatin ;

(b) adding a sodium sulfate solution to the mixture obtained in step a ; and

(c) titrating the solution, obtained in step b with a suitable acid or base titrant while keeping the solution under constant agitation which results in a suspension of encapsulated pharmaceutically active small particles and coacervation of the gelatin.

The suspension from step c is then poured into a cold sodium sulfate solution and stirred at the temperature of an ice bath. This procedure causes « hardening » of the liquid gelatin shell of the microcapsules. The microcapsules are then collected, for instance, by centrifugation.

The ratio of pharmaceutically active compounds to cetyltrimethylammonium bromide to gelatin is about 1 : 0. 1 : 2. The sodium sulfate solution is preferably a 20 % aqueous solution of sodium sulfate.

## Example 2

An aqueous solution consisting of 10 ml of 0.1N sodium sulfadiazine (27.2 g/l), 1 ml of 10 % cetyltrimethylammonium bromide and 10 ml of 10 % gelatin (type B : acid processed) was mixed and maintained at 40-45 °C under constant agitation (medium speed) with a magnetic stirrer for 3 minutes. With constant stirring, 12 ml of 20 % sodium sulfate solution was added and mixing continued for another 3 minute period. The stirring rate was increased and 10 ml of 0.1M HCl was added from a fully opened buret. The resulting white suspension of microencapsulated sulfadiazine was stirred for an additional 10 minutes. The microcapsules were « hardened » by pouring the suspensions into 200 ml of cold (5 °C) 7 % sodium sulfate solution and stirred at medium speed for 30 minutes at ice-bath temperature. According to microscopic inspection, the microcapsules were of a size less than 10 μm.

The procedure is further outlined in the diagram below :

```
┌─────────────────────────────────────────────────────────────────┐
│  Mix 0.1 N sodium sulfadiazine, 10% cetyltrimethyl-              │
│  ammonium bromide and 10% gelatin solutions                      │
└─────────────────────────────────────────────────────────────────┘
                                   │
                                   ↓
┌─────────────────────────────────────────────────────────────────┐
│              Add 20% sodium sulfate solution.                    │
└─────────────────────────────────────────────────────────────────┘
                                   │
                                   ↓
┌─────────────────────────────────────────────────────────────────┐
│  Form sulfadiazine particles by acidification                   │
│  (simultaneously encapsulating with gelatin).                    │
└─────────────────────────────────────────────────────────────────┘
                                   │

        Steps above this line performed at 40-45°C,
          which is above the gelling point of gelatin(35°C).
                                   │
                                   ↓
┌─────────────────────────────────────────────────────────────────┐
│  Gel the encapsulating material by pouring the                  │
│  suspension into cold (5°C) sodium sulfate                       │
│  solution.                                                       │
└─────────────────────────────────────────────────────────────────┘
                                   │
                                   ↓
┌─────────────────────────────────────────────────────────────────┐
│  Collect the microcapsules by centrifugation.                    │
└─────────────────────────────────────────────────────────────────┘
```

According to a third embodiment of the invention, the process comprises the following steps which are carried out at room temperature.

(a) mixing a solution of a suitable pharmaceutically active compound and sodium lauryl sulfate ;
(b) adding methylcellulose and sodium carbomethylcellulose solutions to the mixture obtained in step a ;
(c) adding sodium sulfate solution to the solution obtained in step b ; and
(d) titrating the solution obtained in step c with a suitable acid or base titrant while keeping the solution under constant agitation which results in a suspension of encapsulated pharmaceutically active small particles and coacervation of the gelatin.

The suspension is then poured into a cold sodium sulfate solution and stirred at the temperature of an ice bath. This procedure causes « hardening » of the liquid methyl cellulose/carboxymethylcellulose shell of the microcapsules. The microcapsules are then collected, for instance, by centrifugation.

The ratio of pharmaceutically active compounds to sodium lauryl sulfate is about 1 : (0.5-1). In step b preferably 1 % aqueous solution of methylcellulose and carboxymethylcellulose and in step c preferably a 20 % aqueous solution of sodium sulfate is used.

### Example 3

An aqueous solution consisting of 10 ml of 0.1N sodium sulfadiazine (27.2 g/l) and 0.5 g of sodium lauryl sulfate was mixed at room temperature (25 °C) under constant agitation at medium speed using a magnetic stirrer, until the solution became clear. Two sequential additions were then made, each of which was followed by stirring :

(1) 10 ml each of 1 % methylcellulose (400 cps) and 1 % sodium carboxymethylcellulose (medium viscosity) with stirring for 3 minutes, and
(2) 4 ml of 20 % sodium sulfate with stirring for an additional 3 minutes. With continued stirring at a fast rate, 10 ml of 0.1N hydrochloric acid solution was added from a fully opened buret.

The resulting white suspension of microencapsulated sulfadiazine particles were stirred for an additional 10 minutes. The microcapsules were « hardened » by pouring the suspensions into 200 ml of cold (5 °C) 7 % sodium sulfate solution and stirred at medium speed for 30 minutes at the temperature of ice bath. According to microscopic inspection, the microcapsules were of a size less than 10 $\mu$m. The entire procedure is outlined in the diagram below :

(See table page 7)

...

---

Mix 0.1N sodium sulfadiazine solution and 0.5 g sodium lauryl sulfate.

---

$\downarrow$

---

Add 1% methylcellulose and 1% sodium carboxy-methylcellulose solutions.

---

$\downarrow$

---

Add 20% sodium sulfate solution.

---

$\downarrow$

---

Form sulfadiazine particles by acidification (simultaneously encapsulating with methyl-cellulose/carboxymethylcellulose).

---

$\downarrow$

---

Gel·the encapsulating material by pouring the suspension into cold (5°C) sodium sulfate solution.

---

$\downarrow$

---

Collect the microcapsules by centrifugation

---

According to a fourth embodiment of the invention, the process comprises the following steps which are carried out at room temperature.

(a) mixing solutions of a suitable pharmaceutically active compound and cellulose acetate phthalate ;

(b) adding a sodium sulfate solution to the mixture obtained in step a ; and

(c) titrating the solution obtained in step b with a suitable acid or base titrant while being kept under constant agitation which results in a suspension of encapsulated pharmaceutically active small particles and coacervation of the cellulose acetate phthalate.

7

The suspension is then poured into cold sodium sulfate solution and stirred at the temperature of an ice bath. This procedure causes « hardening » of the liquid cellulose acetate phthalate shell of the microcapsules. The microcapsules are then collected, for instance, by centrifugation.

The ratio of pharmaceutically active compound : cellulose acetate phthalate is 3 : 1. In step b, preferably a 20 % aqueous solution of sodium sulfate is used.

## Example 4

An aqueous solution consisting of 10 ml of 0.1N sodium sulfadiazine (27.2 g/l) and 10 ml of alkaline (0.1M NaOH) 1 % cellulose acetate phthalate (Eastman 4642) was stirred at medium speed with a magnetic stirrer until homogenous. 10 ml of 20 % sodium sulfate was then added and the solution stirred for an additional 10 minutes. The last step was the rapid addition of 10 ml of 0.1N hydrochloric acid from a fully opened buret while the solution was under constant fast agitation with the magnetic stirrer. The resulting suspension of microencapsulated sulfadiazine was stirred for an additional 10 minutes. The microcapsules were « hardened » by pouring the suspensions into 200 ml of cold (5 °C) 7 % sodium sulfate solution and stirred at medium speed for 30 minutes at the temperature of an ice bath.

According to microscopic inspection, the microcapsules were of a size less than 10 μm. The entire procedure is outlined in the diagram below :

```
┌──────────────────────────────────────────────────────────────┐
│  Mix 0.1N sodium sulfadiazine and 1% cellulose               │
│  acetate phthalate solutions.                                 │
└──────────────────────────────────────────────────────────────┘
                              │
                              ↓
┌──────────────────────────────────────────────────────────────┐
│              Add 20% sodium sulfate solution                  │
└──────────────────────────────────────────────────────────────┘
                              │
                              ↓
┌──────────────────────────────────────────────────────────────┐
│  Form sulfadiazine particles by acidification                 │
│  (simultaneously encapsulating with cellulose                 │
│  acetate phthalate).                                          │
└──────────────────────────────────────────────────────────────┘
                              │
                              ↓
┌──────────────────────────────────────────────────────────────┐
│  Gel the encapsulating material by pouring the                │
│  suspension into cold (5°C) sodium sulfate                    │
│  solution.                                                    │
└──────────────────────────────────────────────────────────────┘
                              │
                              ↓
┌──────────────────────────────────────────────────────────────┐
│     Collect the microcapsules by centrifugation               │
└──────────────────────────────────────────────────────────────┘
```

According to the fifth embodiment of the invention, the process comprises the following steps which are performed at 60 °C.

(a) dissolving sodium sulfate in an aqueous solution of a suitable pharmaceutically active compound ;

(b) adding a solution of polyvinylpyrrolidone to the solution obtained in step a ;

(c) adding a solution of sodium sulfate to the solution obtained in step b ; and

(d) titrating the solution obtained in step c with a suitable acid or base titrant while keeping the solution under constant agitation which results in a suspension of encapsulated pharmaceutically active small particles and coacervation of the polyvinyl pyrrolidone.

The suspension is then poured into cold sodium sulfate solution and stirred at the temperature of an ice bath. This procedure causes « hardening » of the liquid polyvinylpyrrolidone shell of the microcapsules. The microcapsules are then collected, for instance, by centrifugation.

The ratio of pharmaceutically active compound : sodium sulfate is 1 : 4.

In step b, preferably a 20 % aqueous solution of sodium sulfate is used.

The sodium sulfate solution to which the suspension of particles is added should have a strength of about 7 % and its temperature should be below 15 °C, preferably in the temperature range 0-15 °C.

## Example 5

A solution consisting of 10 ml of 0.1N sodium sulfadiazine (27.2 g/l) and 1 g of sodium sulfate was prepared by heating to 60 °C while under constant agitation at medium speed with a magnetic stirrer. The solution was then maintained at 60 °C, 10 ml of 2 % polyvinylpyrrolidone (average molecular weight, 360,000) was added, the solution stirred for 3 minutes. The stirring speed was changed to fast and 10 ml of 0.1N hydrochloric acid solution added from an open buret. The white suspension of microcapsules were stirred for 10 minutes.

The microcapsules were « hardened » by pouring the suspensions into 200 ml of cold (5 °C) 7 % sodium sulfate solution and stirred at medium speed for 30 minutes at icebath temperature. According to microscopic inspection, the microcapsules were of a size less than 10 µm. The entire procedure is outlined in the diagram below :

(See table page 10)

```
+--------------------------------------------------------------+
|  Dissolve 1 g of sodium sulfate in 0.1N sodium               |
|  sulfadiazine solution by heating to 60°C.                   |
+--------------------------------------------------------------+
```

```
+--------------------------------------------------------------+
|  Add 2% polyvinylpyrrolidone.                                |
+--------------------------------------------------------------+
```

```
+--------------------------------------------------------------+
|  Add 20% sodium sulfate solution.                            |
+--------------------------------------------------------------+
```

```
+--------------------------------------------------------------+
|  Form sulfadiazine particles by acidification                |
|  (simultaneously encapsulating with polyvinyl-               |
|  pyrrolidone).                                               |
+--------------------------------------------------------------+
```

Steps above this line performed at 60°C

```
+--------------------------------------------------------------+
|  Gel the encapsulating material by pouring the               |
|  suspension into cold (5°C) sodium sulfate solution.         |
+--------------------------------------------------------------+
```

```
+--------------------------------------------------------------+
|  Collect the microcapsules by centrifugation                 |
+--------------------------------------------------------------+
```

According to the sixth embodiment, the process comprises the following steps which are carried out at a temperature above 35 °C, preferably 38 °C.

(a) dissolving gelatin in water ;
(b) adding sodium sulfate to the solution from step a ;
(c) adding a suitable pharmaceutically active compound in the solution from the step b ;
(d) adding a sodium hydroxide solution or other alkaline solution, for example, potassium hydroxide, or adding a hydrochloric acid solution or other acidic solution, for example, acetic acid, to the solution from step c ; and
(e) titrating the solution obtained in step (d) with a suitable amount of acid or base titrant while keeping the solution under constant agitation which results in a suspension of encapsulated pharmaceutically active small particles and coacervation of the gelatin ; and
(f) the suspension is then poured into cold sodium sulfate solution and stirred at the temperature of an ice bath to cause « gelling » of the liquid gelatin shell of the microcapsules, and the microcapsules are collected, for instance, by centrifugation ; or

(g) the suspension in cold sodium sulfate solution is centrifuged and washed twice with water, centrifuged, dispersed into water, formaldehyde solution added under stirring and the suspension allowed to stand at room temperature. This procedure causes hardening of the gelled microcapsule shell. The suspension is centrifuged, the microcapsules washed twice with water, redispersed in water with stirring, isopropanol added, filtered, washed twice with isopropanol, filtered and dried. This procedure causes dehydration of the hardened microcapsules. Alternatively the microcapsules from step f collected directly, for instance, by centrifugation, are redispersed in cold water (5 °C), a solution consisting of formaldehyde and isopropanol is added, the suspension allowed to stand at room temperature, and then collected, for instance, by centrifugation. The ratio of pharmaceutically active compound to sodium sulfate is, for example, 1 : 2. The gelatin solution should preferably be prepared from type B (acid processed) gelatin, and of a pharmaceutical grade. The gelatin should be added as a 2-10 % (w/w) solution, preferably a 5 % (w/w) solution. The formaldehyde should be added as a 5-37 % (w/w) solution, preferably a 37 % (w/w) solution. The alcohol can be any water-miscible alcohol, preferably isopropanol, and the mixture with water can be 5-50 % (w/w) isopropanol. Alternatively, a formaldehyde-alcohol mixture can be used as a 1 : 5-30 (v/v) mixture (formaldehyde, 38 % : alcohol), preferably a 1 : 19 (v/v) mixture (formaldehyde, 38 % : isopropanol).

## Example 6

A solution consisting of 0.34 g of gelatin (type B : acid processed) in 20 ml of water was prepared, 1.34 g of sodium sulfate was added, followed by 1.0 g of 9-(3.4-dihydroxybutyl)-guanine and then by 1.0 ml of 5.0 N sodium hydroxide solution, and the resulting solution was titrated while under constant agitation with a magnetic stirrer, with 0.95 ml of 5.0 N hydrochloric acid solution. This procedure resulted in a white suspension of microencapsulated 9-(3.4-dihydroxybutyl)-guanine particles. The suspension was then stirred at 38 °C for 3 minutes and then at room temperature for an additional 20 minutes, following which the microcapsules were collected by centrifugation. The microcapsules were redispersed into 0.5 ml of cold water, 20 ml of a solution consisting of formaldehyde solution 38 %, and isopropanol in the ratio of 1 : 19 (v/v) was added under stirring and the suspension allowed to stand at room temperature for 15-20 hours. The suspension was centrifuged and the microcapsules collected and dried in a low pressure oven at 35 °C. This procedure caused hardering and dehydration of the microcapsules. The dry microcapsules were stored in wellclosed containers at room temperature. The entire process was monitored by observation of samples in the optical microscope. The microcapsules were of assymetric appearance and of a size less than 10 μm.

A schematic diagram of the entire process according to Example 6 is illustrated below :

(See table page 12)

```
+----------------------------------------------------------+
|  Mix 1.7% gelatin solution with 6.7% sodium sulfate and  |
|  5% 9-(3,4-dihydroxybutyl)-guanine.                      |
+----------------------------------------------------------+
```

```
+----------------------------------------------------------+
|                                                          |
|  Add 5% 5N sodium hydroxide solution.                    |
+----------------------------------------------------------+
```

```
+----------------------------------------------------------+
|  Form 9-(3,4-dihydroxybutyl)-guanine particles by acidification |
|  (simultaneously encapsulating with gelatin).            |
+----------------------------------------------------------+
```

Steps above this line performed at 38°C,
which is above the gelling point of gelatin (35°C)

```
+----------------------------------------------------------+
|  Harden the encapsulating material and dehydrate the     |
|  microcapsules by adding a solution of formaldehyde in   |
|  isopropanol.                                            |
+----------------------------------------------------------+
```

```
+----------------------------------------------------------+
|  Collect the microcapsules by centrifugation.            |
+----------------------------------------------------------+
```

## Claims

1. A process for encapsulating a weakly acidic organic compound whose solubility in water is greater at a first pH than at a second pH which process comprises :
(a) dissolving said compound in water in the presence of sufficient base to raise the pH to said first pH and at least 2 pH units above the pKa of the compound, together with an encapsulating material and an electrolyte which is effective, but present in an amount just insufficient to cause coacervation of the encapsulating material without interacting with it ;
(b) stirring and titrating the solution with a titrant effective to reduce the pH of said solution to said second pH to cause the concurrent precipitation of the compound as small particles and formation of a coacervate of the encapsulating material ; and
(c) gelling the encapsulating material.
2. A process for encapsulating a weakly basic organic compound whose solubility in water is greater at a first pH than at a second pH which process comprises :
(a) dissolving said compound in water in the presence of sufficient acid to lower the pH to said first pH and at least 2 pH units below the pKa of the compound, together with an encapsulating material and an amount of an electrolyte which is effective, but just insufficient to cause coacervation of the encapsulating material without interacting with it ;

(b) stirring and titrating the solution with a titrant effective to raise the pH of said solution to said second pH to cause the concurrent precipitation of the compound as small particles and formation of a coacervate of the encapsulating material ; and

(c) gelling the encapsulating material.

3. A process according to claims 1 or 2 wherein the compound is pharmaceutically active.

4. A process according to claim 2 wherein the compound is sulfadiazine in sodium sulfate solution containing ethyl alcohol and the encapsulating material is a gelatin solution.

5. The process according to claim 3 wherein the first pH is an acid pH.

6. A process according to claim 1 or 2 wherein a wetting agent is used in step a.

7. A process according to claim 1 or 2 wherein, the temperature is controlled in step b.

8. A process according to claim 1, wherein the pharmaceutically active compound is selected from the group consisting of bacampicillin, griseofulvin, indomethacin, sodium sulfadiazine, erythromycin, theophylline, salicylic acid, acetylsalicylic acid, chlorozoxazone, lidocaine and alaproclate.

9. A process according to claim 1, wherein the encapsulating material is selected from the group consisting of gelatin, methylcellulose, sodium carboxymethylcellulose, cellulose acetate phthalate, and polyvinylpyrrolidone.

10. A process according to claim 4, wherein the ratio of compound to encapsulating material to wetting agent to electrolyte is about (0.1-6) : (0.1-4) : (0.1-10) : (0.4-48).


## Patentansprüche

1. Verfahren zur Verkapselung einer schwach sauren organischen Verbindung, deren Löslichkeit in Wasser bei einem ersten pH-Wert größer ist als bei einem zweiten pH-Wert, welches Verfahren umfaßt :

(a) Lösung der Verbindung in Wasser in Anwesenheit ausreichender Base zur Anhebung des pH-Werts auf den ersten pH-Wert und zumindest 2 pH-Einheiten über den pKa der Verbindung, zusammen mit einem Verkapselungsmaterial und einem Elektrolyten, der wirksam ist, doch in einer Menge vorhanden ist, die gerade nicht ausreicht, um eine Koazervierung des Verkapselungsmaterials zu verursachen, ohne mit diesem in Wechselwirkung zu treten ;

(b) Rühren und Titrieren der Lösung mit einem Titrans, das die Reduzierung des pH-Werts der Lösung zum zweiten pH-Wert bewirkt, um die gleichzeitige Ausfällung der Verbindung als kleine Teilchen und die Bildung eines Koazervats des Verkapselungsmaterials zu bewirken ; und

(c) Gelieren des Verkapselungsmaterials.

2. Verfahren zur Verkapselung einer schwach basischen organischen Verbindung, deren Löslichkeit in Wasser bei einem ersten pH-Wert größer ist als bei einem zweiten pH-Wert, welches Verfahren umfaßt :

(a) Lösung der Verbindung in Wasser in Anwesenheit ausreichender Säure zur Senkung des pH-Werts auf den ersten pH-Wert und zumindest 2 pH-Einheiten unter den pKa der Verbindung, zusammen mit einem Verkapselungsmaterial und einer Menge eines Elektrolyten, die wirksam ist, doch gerade nicht ausreicht, um eine Koazervierung des Verkapselungsmaterials zu bewirken, ohne mit diesem in Wechselwirkung zu treten ;

(b) Rühren und Titrieren der Lösung mit einem Titrans, das die Anhebung des pH-Werts der Lösung auf den zweiten pH-Wert bewirkt, um die gleichzeitige Ausfällung der Verbindung als kleine Teilchen und die Bildung eines Koazervats des Verkapselungsmaterials zu bewirken ; und

(c) Gelieren des Verkapselungsmaterials.

3. Verfahren nach Anspruch 1 oder 2, worin die Verbindung pharmazeutisch aktiv ist.

4. Verfahren nach Anspruch 2, worin die Verbindung Sulfadiazin in Natriumsulfatlösung enthaltend Äthylalkohol ist und das Verkapselungsmaterial eine Gelatinelösung ist.

5. Verfahren nach Anspruch 3, worin der erste pH-Wert ein saurer pH-Wert ist.

6. Verfahren nach Anspruch 1 oder 2, worin ein Netzmittel in Schritt a verwendet wird.

7. Verfahren nach Anspruch 1 oder 2, worin die Temperatur in Schritt b gesteuert wird.

8. Verfahren nach Anspruch 1, worin die pharmazeutisch aktive Verbindung ausgewählt ist aus der Gruppe bestehend aus Bacampicillin, Griseofulvin, Indomethacin, Natriumsulfadiazin, Erythromycin, Theophyllin, Salicylsäure, Acetylsalicylsäure, Chlorozoxazon, Lidocain und Alaproclat.

9. Verfahren nach Anspruch 1, worin das Verkapselungsmaterial ausgewählt ist aus der Gruppe bestehend aus Gelatine, Methylcellulose, Natriumcarboxymethylcellulose, Celluloseacetatphthalat und Polyvinylpyrrolidon.

10. Verfahren nach Anspruch 4, worin das Verhältnis der Verbindung zum Verkapselungsmaterial zum Netzmittel zum Elektrolyten etwa (0,1-6) : (0,1-4) : (0,1-10) : (0,4-48) ist.


## Revendications

1. Un procédé pour l'encapsulation d'un composé organique faiblement acide dont la solubilité dans l'eau à un premier pH est supérieure à celle à un second pH, procédé qui consiste :

(a) à dissoudre ledit composé dans de l'eau en présence de suffisamment de base pour élever le pH jusqu'au premier pH et à au moins 2 unités de pH au-dessus du pKa du composé, conjointement avec une substance d'encapsulation et un électrolyte qui est efficace, mais présent en une quantité juste insuffisante pour provoquer la coacervation de la substance d'encapsulation sans interagir avec elle ;

(b) à agiter et à titrer la solution avec un agent de titrage efficace pour réduire le pH de ladite solution jusqu'au second pH en provoquant la précipitation simultanée du composé sous forme de fines particules et la formation d'un coacervat de la substance d'encapsulation, et

(c) à gélifier la substance d'encapsulation.

2. Un procédé pour l'encapsulation d'un composé organique faiblement basique dont la solubilité dans l'eau à un premier pH est supérieure à celle à un second pH, procédé qui consiste :

(a) à dissoudre ledit composé dans de l'eau en présence de suffisamment d'acide pour abaisser le pH jusqu'au premier pH et à au moins 2 unités de pH au-dessous du pKa du composé, conjointement avec une substance d'encapsulation et une quantité d'un électrolyte qui est efficace, mais juste insuffisant pour provoquer la coacervation de la substance d'encapsulation sans interagir avec elle ;

(b) à agiter et à titrer la solution avec un agent de titrage efficace pour élever le pH de ladite solution jusqu'au second pH en provoquant la précipitation simultanée du composé sous forme de fines particules et la formation d'un coacervat de la substance d'encapsulation, et

(c) à gélifier la substance d'encapsulation.

3. Procédé selon la revendication 1 ou 2, dans lequel le composé est pharmaceutiquement actif.

4. Procédé selon la revendication 2, dans lequel le composé est de la sulfadiazine en solution avec du sulfate de sodium contenant de l'alcool éthylique et la substance d'encapsulation est une solution de gélatine.

5. Procédé selon la revendication 3, dans lequel le premier pH est un pH acide.

6. Procédé selon la revendication 1 ou 2, dans lequel un agent mouillant est utilisé dans l'étape    a).

7. Procédé selon la revendication 1 ou 2, dans lequel la température est réglée dans l'étape    b).

8. Procédé selon la revendication 1, dans lequel le composé pharmaceutiquement actif est choisi dans le groupe composé de bacampicilline, de griséofulvine, d'indométhacine, de sulfadiazine sodique, d'érythromycine, de théophylline, d'acide salicylique, d'acide acétylsalicylique, de chlorozoxazone, de lidocaïne et d'alaproclate.

9. Procédé selon la revendication 1, dans lequel la substance d'encapsulation est choisie dans le groupe composé de gélatine, de méthylcellulose, de carboxyméthylcellulose de sodium, d'acéto-phtalate de cellulose et de polyvinylpyrrolidone.

10. Procédé selon la revendication 4, dans lequel le rapport entre le composé, la substance d'encapsulation, l'agent mouillant et l'électrolyte est d'environ (0,1-6) : (0,1-4) : (0,1-10) : (0,4-48).